# EUROPEAN PATENT APPLICATION

(11) **EP 4 674 432 A1**
(43) Date of publication of application: **07.01.2026**
(21) Application number: 24763225.0
(22) Date of filing: 29.02.2024
(51) Int. Cl.: A61K 45/06, A61K 39/00, C07K 19/00, A61P 35/00

(54) **COMBINATION DRUG CONTAINING CD20/CD47 BLOCKING BIFUNCTIONAL FUSION PROTEIN, AND USE THEREOF**

(30) Priority: 01.03.2023 CN 202310194436
(71) Applicant: Shanghai JMT-Bio Technology Co., Ltd., Shanghai 200032 (CN)
(72) Inventor: SONG, Liping, Shanghai 200032 (CN); YE, Wenrui, Shanghai 200032 (CN); FAN, Yi, Shanghai 200032 (CN); HUANG, Xiaohui, Shanghai 200032 (CN); MU, Jun, Shanghai 200032 (CN)
(74) Representative: Mewburn Ellis LLP
(86) International application number: PCT/CN2024/079347
(87) International publication number: WO 2024/179545

(57) **Abstract**

Provided are a method and a use for treating cancers by using a CD20/CD47 double-blocking bifunctional fusion protein in combination with a bifunctional alkylating agent, in particular a method and a use for treating cancers (especially non-Hodgkin lymphoma) by using a CD20/CD47 double-blocking bifunctional fusion protein in combination with bendamustine or a salt thereof.

## Description

### RELATED APPLICATIONS

This application claims priority to Chinese Patent Application No. 202310194436.8, filed on March 1, 2023, entitled "COMBINATION DRUG CONTAINING CD20/CD47 BLOCKING BIFUNCTIONAL FUSION PROTEIN, AND USE THEREOF", which is herein incorporated by reference in its entirety for all purposes.

### TECHNICAL FIELD

The present application relates to the field of biopharmaceuticals. More specifically, the present application relates to a method and use for treating cancers by using a CD20/CD47 double-blocking bifunctional fusion protein in combination with a bifunctional alkylating agent, in particular a method and use for treating cancers (especially non-Hodgkin lymphoma) by using a CD20/CD47 double-blocking bifunctional fusion protein in combination with bendamustine or a salt thereof.

### BACKGROUND ART

Non-Hodgkin lymphoma (NHL) is mostly of the B-cell type, accounting for 70%-85% of the total. There are mainly four kinds of B cell types: diffuse large B-cell lymphoma (DLBCL), follicular lymphoma (FL), mantle cell lymphoma (MC), and mucosa-associated lymphoid tissue lymphoma (MAL). Among them, diffuse large B-cell lymphoma is the most common. Clinically, lymphadenopathy is the most common local clinical manifestation. In recent years, the incidence of lymphoma in China has gradually increased, and the current incidence is nearly 7/100,000 people per year.

Currently, R-CHOP (a combination therapy consisting of 5 drugs, in which R represents Rituxan (Rituximab injection), C represents cyclophosphamide, H represents doxorubicin, O represents vincristine, and P represents prednisone) is the first-line drug for the treatment of patients with non-Hodgkin's lymphoma such as diffuse large B-cell lymphoma. Compared with the traditional CHOP regimen, the addition of rituximab in R-CHOP regimen increases the 5-year median overall survival of the patients from 45% to 58%, indicating that the immunochemotherapy regimen can further improve the therapeutic effects on the patients based on the original chemotherapy regimen.

The action mechanisms of rituximab include CDC (complement-dependent cytotoxicity), ADCC (antibody-dependent cell-mediated cytotoxicity) and ADCP (antibody-dependent cell-mediated phagocytosis), among which, CDC plays a major role.

The reasons for resistance or recurrence of non-Hodgkin's lymphoma after treatment with R-CHOP may involve either the four chemotherapeutic drugs of CHOP or rituximab.

Bendamustine is a bifunctional alkylating agent that can overcome the cross-resistance of previous alkylating agents (such as cyclophosphamide). Bendamustine in combination with rituximab has been developed and approved for the second-line treatment of non-Hodgkin's lymphoma (NHL), achieving a good therapeutic effect.

At the same time, CD47 has been found to be a new immune checkpoint that can bind to the SIRPα protein on macrophages, thereby preventing the phagocytosis of macrophages. Blocking the binding of CD47 to SIRPα can promote the phagocytosis of macrophages. Currently, several anti-CD47 monoclonal antibodies have been developed. Studies have further shown that the combination of an anti-CD47 monoclonal antibody and rituximab can further enhance the phagocytosis of macrophages. However, existing treatment regimens still cannot meet the treatment needs of relevant patients.

### DESCRIPTION OF THE INVENTION

In previous studies, the inventors of the present application developed a new generation of CD20/CD47 double-blocking bifunctional fusion protein, which significantly outperforms SIRPα-Fc fusion proteins in blocking the interaction of CD47 on tumor cells with SIRPα expressed on macrophages. Further studies by the inventors have shown that the CD20/CD47 double-blocking bifunctional fusion protein has a stronger phagocytic activity of macrophages than rituximab, SIRPα fusion protein, and ofatumumab, etc.; and can also produce a significant therapeutic effect in a rituximab-resistant diffuse large B-cell lymphoma (DLBCL) xenograft model.

On this basis, the inventors of the present application have discovered and provided a combination drug for preventing or treating cancer through extensive research and exploration, including a CD20/CD47 double-blocking bifunctional fusion protein and a bifunctional alkylating agent, especially the bifunctional alkylating agent bendamustine (including bendamustine hydrochloride), as well as a use of the combination drug in the treatment of cancer and the manufacture of a medicament for treating cancer. In particular, it was found that when the combination of the CD20/CD47 double-blocking bifunctional fusion protein and a bifunctional alkylating agent, especially the bifunctional alkylating agent bendamustine (including bendamustine hydrochloride) is administrated for the treatment of lymphoma, especially diffuse large B-cell lymphoma, a very significant synergistic effect is shown.

Specifically, the present application provides a pharmaceutical composition for preventing or treating cancer, comprising a CD20/CD47 double-blocking bifunctional fusion protein and a bifunctional alkylating agent.

The terms "pharmaceutical composition", "combination drug", "drug combination", "drugs used in combination" and the like used herein are used interchangeably to denote a combination of at least one drug and, optionally, a pharmaceutically acceptable carrier or excipient combined together to achieve a certain specific purpose. In certain embodiments, the pharmaceutical composition includes a combination that is separated in time and/or space, as long as it can work together to achieve the purpose of the present application. For example, the components contained in the pharmaceutical composition (e.g., the fusion protein and the bifunctional alkylating agent according to the present application) can be administered to a subject as a whole or can be separately administered to a subject. When the components contained in the pharmaceutical composition are separately administered to a subject, the components can be administered to the subject simultaneously or sequentially. For example, the pharmaceutically acceptable carrier can be water, buffered aqueous solution, isotonic saline solution such as PBS (phosphate buffered saline), glucose, mannitol, dextrose, lactose, starch, magnesium stearate, cellulose, magnesium carbonate, 0.3% glycerol, hyaluronic acid, ethanol or polyalkylene glycols such as polypropylene glycol, triglycerides, or the like. The type of pharmaceutically acceptable carrier used depends, *inter alia,* on whether the composition according to the present application is formulated for administration by inhalation, intranasal route, oral route, intravenous injection, subcutaneous injection or intramuscular injection. The composition according to the present application can comprise wetting agents, emulsifying agents or buffer substances as additives.

The cancers/tumors treated by the present application include, but are not limited to, lymphoma, breast cancer, colorectal cancer, lung cancer, pancreatic cancer, esophageal cancer, endometrial cancer, ovarian cancer, gastric cancer, prostate cancer, kidney cancer, cervical cancer, myeloma, leukemia, thyroid cancer, uterine cancer, bladder cancer, neuroendocrine cancer, head and neck cancer, liver cancer, nasopharyngeal cancer, testicular cancer, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma or myelodysplastic syndrome. In some embodiments, the cancer is a CD20-positive tumor. In some embodiments, the cancer is lymphoma. In some embodiments, the cancer is Hodgkin lymphoma or non-Hodgkin lymphoma. In some embodiments, the cancer is non-Hodgkin's lymphoma. In some embodiments, the cancer is diffuse large B-cell lymphoma, follicular lymphoma, mantle cell lymphoma, or mucosa-associated lymphoid tissue lymphoma. In some embodiments, the cancer is diffuse large B-cell lymphoma. In some embodiments, the cancer is CD20-positive non-Hodgkin lymphoma. In some embodiments, the cancer is CD20-positive diffuse large B-cell lymphoma.

The CD20/CD47 double-blocking bifunctional fusion protein described herein comprises two functional moieties that block CD20 and CD47, respectively. In some examples of the present application, the two functional moieties are also referred to as two "arms", which can form a heterodimer in the form of a homodimer structure similar to a natural antibody. It will be appreciated by those skilled in the art that the bifunctional fusion protein of the present application is not limited to the two-arm form, but may also be a four-arm form or other forms, similar to various configurations of bispecific antibodies in the art.

The term "antibody" as used herein broadly encompasses any form of molecule that can specifically bind to an antigen, and can include an intact antibody and antigen-binding fragment ("antigen-binding portion") or single-chain form thereof. The term "antigen binding portion" or "antigen binding fragment" may particularly refer to antibody fragments, such as Fv, scFv, Fab, F(ab')₂, Fab', VHH, etc.

In some embodiments, the functional portion/arm that blocks CD20 is in the form of an antibody or antigen binding portion thereof against CD20.

In some embodiments, the functional portion/arm that blocks CD47 is an antibody or antigen-binding portion thereof against CD47 and/or a natural ligand of CD47 or a ligand active moiety (wild type or mutant) derived from the natural ligand. In some embodiments, the natural ligand of CD47 is SIRPα protein. The SIRPα protein is a transmembrane protein, and therefore, its ligand activity function is mainly performed by the extracellular domain. Therefore, in some embodiments, the ligand active moiety of the SIRPα protein comprises the entire extracellular domain of the SIRPα protein. In some embodiments, the ligand active moiety of the SIRPα protein is an extracellular truncation of the SIRPα protein. In some embodiments, the ligand active moiety of the SIRPα protein is the extracellular D1 domain of the SIRPα protein (e.g., the amino acid sequence shown at positions 20-136 of SEQ ID NO:6) or a non-high affinity mutant thereof (e.g., the amino acid sequence shown at positions 20-136 of SEQ ID NO:3, which comprises a N99A mutation with respect to SEQ ID NO:6 (i.e., a N80A mutation with respect to the extracellular D1 domain itself)). In some embodiments, it is more desirable that the functional portion/arm that blocks CD47 has a lower affinity for CD47, and therefore, the non-high affinity mutants (i.e., other mutations of interest that do not result in increased affinity) are desirable. In addition to the extracellular D1 domain of SIRPα protein, SEQ ID NO: 3 comprises a signal peptide and a Fc region for forming a dimer with SEQ ID NO: 1, as described in detail below.

In some embodiments of the two-arm form of the CD47/CD20 dual-targeting fusion protein, the first arm targets CD20 with high affinity, and the second arm blocks the interaction of CD47 and SIRPα with low affinity. In some specific embodiments, the first arm functions as an antibody, and the second arm functions as a natural ligand of CD47 or a ligand active moiety derived from the natural ligand, such as an extracellular truncation of the SIRPα protein or a non-high affinity mutant thereof. The "high affinity" and "low affinity" described herein describes the relative relationship of the affinities of the dual-target fusion protein of the present application to CD47 protein and CD20 protein. For example, the affinity levels of the dual-target fusion protein to the CD47 protein and the CD20 protein can be measured at the protein level, e.g., by determining Kd values, and the affinities can be determined by comparing the Kd values. In some specific embodiments, the binding affinity of the CD47/CD20 dual-target fusion protein to the CD20 protein is at least 6 times that of the CD47/CD20 dual-target fusion protein to the CD47 protein. In some embodiments, the two-arm form of the CD47/CD20 dual-target fusion protein is in the form of left and right arms similar to a natural antibody (see the general schematic diagram of antibody structure, as distinguished from possible upper and lower arm structures), and the left and right arms are also used to describe such embodiments below. In some embodiments, the left arm targets CD20 with high affinity and the right arm blocks the interaction of CD47 with SIRPα with low affinity.

In some embodiments, the first arm and/or the second arm is in the form of an immunoglobulin Fab or Fab'. In some embodiments, the first left or left arm is in the form of an immunoglobulin Fab or Fab', and the second arm or right arm comprises an extracellular truncation of SIRPα, or a non-high affinity mutant thereof. In some embodiments, the first arm or left arm comprises the amino acid sequence shown at positions 20-244 of SEQ ID NO:1 and the amino acid sequence shown at positions 21-234 of SEQ ID NO:2; and the second arm or right arm comprises the amino acid sequence shown at positions 20-136 of SEQ ID NO:3 or 6. In some embodiments, the first arm or left arm comprises the amino acid sequence shown at positions 20-241 of SEQ ID NO:4 and the amino acid sequence shown at positions 21-239 of SEQ ID NO:5; and the second arm or right arm comprises the amino acid sequence shown at positions 20-136 of SEQ ID NO:3 or 6. SEQ ID NOs: 1 and 2 respectively show the heavy chain and light chain amino acid sequences (with signal peptides, respectively) of one exemplary antibody targeting CD20. SEQ ID NOs: 4 and 5 respectively show the heavy chain and light chain amino acid sequences (with signal peptides, respectively) of another exemplary antibody targeting CD20.

In some embodiments, the bifunctional fusion protein further comprises an Fc region. In some embodiments, the two arms of the bifunctional fusion protein each comprise an Fc region, and form a heterodimer by means of the interaction of the Fc regions. In some embodiments, the Fc region is wild-type. In some embodiments, a Knobs-into-holes mutation is present in the Fc region. The knobs-into-holes mutation in the Fc region is a commonly used technique in the art for improving dimer assembly. As an example, the Fc region comprised in each of SEQ ID NO: 1 and SEQ ID NO: 3 comprises engineering modification to achieve Knobs-into-holes. In some embodiments, the first arm or left arm of the bifunctional fusion protein comprises the amino acid sequence shown at positions 20-471 of SEQ ID NO:1 and the amino acid sequence shown at positions 21-234 of SEQ ID NO:2; and the second arm or right arm comprises the amino acid sequence shown at positions 20-363 of SEQ ID NO:3 or 6. In some embodiments, the first arm or left arm of the bifunctional fusion protein comprises the amino acid sequence shown at positions 20-468 of SEQ ID NO:4 and the amino acid sequence shown at positions 21-239 of SEQ ID NO:5; and the second arm comprises the amino acid sequence shown at positions 20-363 of SEQ ID NO:3 or 6. As shown in the sequence structures of SEQ ID NOs:1-6 listed at the end of the specification, positions 1-19 of SEQ ID NO:1, positions 1-20 of SEQ ID NO:2, positions 1-19 of SEQ ID NO:3, positions 1-19 of SEQ ID NO:4, positions 1-20 of SEQ ID NO:5, and positions 1-19 of SEQ ID NO:6 are signal peptides, which may be replaced by signal peptides of other structures or omitted.

In some embodiments, the first arm or left arm comprises the amino acid sequences as set forth in SEQ ID NO:1 and SEQ ID NO:2; and the second arm or right arm comprises the amino acid sequence as set forth in SEQ ID NO:3.

The bifunctional alkylating agent described in the present application is a bifunctional derivative of nitrogen mustard. In some embodiments, the bifunctional alkylating agent is bendamustine or a pharmaceutically acceptable salt, solvate or prodrug thereof; chlorambucil; or L-phenylalanine mustard. In some embodiments, the bifunctional alkylating agent is bendamustine hydrochloride.

The present application further provides use of the above-described CD20/CD47 double-blocking bifunctional fusion protein and bifunctional alkylating agent in the manufacture of a pharmaceutical composition for treating cancer (preferably, a CD20-positive tumor).

The present application further provides a combination comprising the above-described CD20/CD47 double-blocking bifunctional fusion protein and bifunctional alkylating agent for use in the treatment of cancer (preferably, a CD20-positive tumor).

The present application further provides a method for treating cancer (preferably, a CD20-positive tumor) in an individual, the method comprising administering to the individual in need thereof a therapeutically effective amount of the above-described CD20/CD47 double-blocking bifunctional fusion protein and bifunctional alkylating agent.

In some embodiments of the above use or combination or method, the CD20/CD47 double-blocking bifunctional fusion protein and the bifunctional alkylating agent are formulated together or are physically separate (e.g., as different units of a kit or medical kit). The CD20/CD47 double-blocking bifunctional fusion protein can be formulated for administration before, simultaneously with, or after the bifunctional alkylating agent.

In some embodiments, the bifunctional fusion protein is administered at a dose of 0.3 mg/kg-20 mg/kg, for example, 0.5 mg/kg, 2 mg/kg, 5 mg/kg, 10 mg/kg or 15 mg/kg. In some embodiments, the bifunctional fusion protein is administered at a frequency of at least once every 2 weeks, for example, once every 2 weeks, once every 3 weeks, or once every 4 weeks, preferably once every 2 weeks or once every 3 weeks; alternatively, the administration frequency may also be multiple times a week, such as twice a week.

In some embodiments, the bifunctional alkylating agent (e.g., bendamustine (including bendamustine hydrochloride)) is administered at a dose of 5 mg/kg-40 mg/kg, for example, 5 mg/kg, 10 mg/kg, 20 mg/kg, 30 mg/kg or 40 mg/kg. In some embodiments, the bifunctional alkylating agent (e.g., bendamustine (including bendamustine hydrochloride)) is administered at a frequency of at least once every 2 weeks, for example, once every 2 weeks, once every 3 weeks, or once every 4 weeks, preferably once every 2 weeks or once every 3 weeks; alternatively, the administration frequency may also be multiple times a week, for example, twice a week.

In some embodiments, the CD20/CD47 double-blocking bifunctional fusion protein and the bifunctional alkylating agent are independently administered by inhalation, intranasal route, oral route, intravenous injection, subcutaneous injection or intramuscular injection.

Examples of the CD20/CD47 double-blocking bifunctional fusion protein described in the present application can be found in Chinese Patent Application No. 201810430371.1 (CN 108864290 B), which is incorporated herein by reference in its entirety.

One example of the CD20/CD47 double-blocking bifunctional fusion protein described in the present application comprises a left arm consisting of the amino acid sequences as set forth in SEQ ID NO:1 and SEQ ID NO:2 that targets CD20 with high affinity, and a right arm with the amino acid sequence as set forth in SEQ ID NO:3 that blocks the interaction between CD47 and SIRPα with low affinity. The CD20/CD47 double-blocking bifunctional fusion protein is referred to as JMT601 in the present application. Another example of the CD20/CD47 double-blocking bifunctional fusion protein described in the present application comprises a left arm consisting of the amino acid sequences as set forth in SEQ ID NO: 4 and SEQ ID NO: 5 that targets CD20 with high affinity, and a right arm with the amino acid sequence as set forth in SEQ ID NO: 3 or 6 that blocks the interaction between CD47 and SIRPα with low affinity.

The preparation of JMT601 can refer to the preparation methods disclosed in Examples 1 and 2 of CN 108864290 B, or can be prepared by other methods in the prior art.

### BRIEF DESCRIPTION OF THE DRAWINGS

FIG. 1 shows the tumor volume change curves in each treatment group of the WSU-DLCL2 human lymphoma subcutaneous model. Note: Data are expressed as "mean ± standard error".
FIG. 2 shows the changes in body weight of experimental mice in each treatment group of the WSU-DLCL2 human lymphoma subcutaneous model over the course of treatment. Note: Data are expressed as "mean ± standard error".

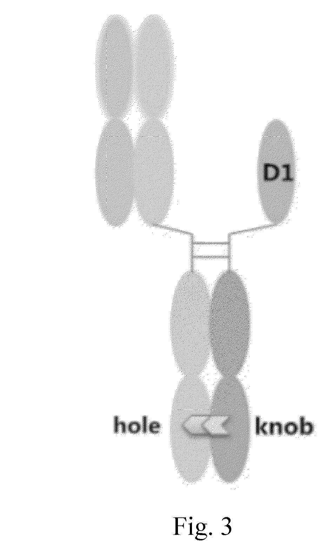

FIG. 3 shows a schematic structural diagram of one embodiment of the CD20/CD47 double-blocking bifunctional fusion protein of the present application, a representative example of which is JMT601. The left arm is a single-arm form of a natural antibody targeting CD20, and the Fc region comprises a hole mutation; the right arm is the extracellular D1 domain of the SIRPα protein, and the Fc region comprises a knob mutation.

### Examples

### Example 1: Pharmacodynamic study of combined drug therapy in WSU-DLCL2 human lymphoma subcutaneously transplanted NOD SCID mouse model

### 1. Experimental Materials and Objects

The main experimental materials used in the experiment were as follows.

**Table 1: Main experimental materials and reagents used**

| Experimental material or data | Relevant information |
|---|---|
| NOD SCID mice | Age: 7-9 weeks; Sex: Female, Shanghai Lingchang Biotechnology Co., Ltd. Feeding environment: SPF grade |
| RPM11640 | Gibco Batch No.: 8119467 |
| Fetal bovine serum | Gibco Batch No.: 2120135CP |
| Matrigel | Corning Batch No.: 9231006 |
| Electric aspirator | Thermo scientific; Model No.: T01856 S1 |
| Pipette | RANIN; Model No.: 100µL-1000µL, 20µL-200µL |
| JMT601 | JMT-BIO Technology Batch No.: JMT60120200129, Concentration: 20 mg/ml |
| Rituxan (Rituximab) | Roche Batch No.: H0269, Concentration: 10 mg/ml |
| Bendamustine hydrochloride | Meilun Batch No.: D0201A, Concentration: salt factor:1.102 |
| IgG1 control (hIgG1) | Crownbio Cat. No.: AB190055, Concentration: 7.6mg/ml |

In this study, the WSU-DLCL2 human lymphoma subcutaneous transplant model was used to evaluate the efficacy of a combination of the test drugs in NOD SCID mice. The design strategy for the pharmacodynamic test was outlined below.

**Table 2: Dosage regimen**

| **Group** | **N** | **Dosing group** | **Administration Dosage (mg/kg)** | **Mode of Administration** | **Administration Duration** |
|---|---|---|---|---|---|
| 1 | 8 | IgG1 control | 10 | *i*.*v*. | BIW: 3 weeks |
| 2 | 8 | Rituximab | 10 | *i.v*. | BIW: 3 weeks |
| 3 | 8 | JMT601 | 3.3 | *i.v.* | BIW**:** 3 weeks |
| 4 | 8 | JMT601 | 10 | *i.v*. | BIW**:** 3 weeks |
| 5 | 8 | Bendamustine | 30 | *i.v*. | single administration |
| 6 | 8 | JMT601 | 3.3 | *i.v.* | BIW**:** 3 weeks |
| | | Bendamustine | 30 | *i.v*. | single administration |

| | | | | | |
|---|---|---|---|---|---|
| Note: 1. BIW=twice a week, N=number of animals; 2. The dosage volume was 10 µl/g | | | | | |

### 2. Experimental Methods

### 2.1 Tumor inoculation and grouping

WSU-DLCL2 cells (human diffuse large B-cell lymphoma cells) (Cell Numbering: CL-00648, Manufacturer: Crownbio) were cultured in RPMI 1640 medium containing 10% fetal bovine serum. WSU-DLCL2 cells in the exponential growth phase were collected, resuspended in PBS to a suitable concentration, and then were used for subcutaneous tumor inoculation in mice.

1×10⁷ WSU-DLCL2 cells were resuspended in PBS and Matrigel at a ratio of 1:1 (0.1 ml/mouse) and inoculated subcutaneously in experimental mice. The vaccination date was May 20, 2020. When the average tumor volume reached 150.53 mm³, the mice were randomly divided into groups according to tumor size (see Table 2 for details).

### 2.2 Methods for formulating test and control drugs

**Table 3: Pharmaceutical formulation methods and storage conditions**

| **Test drugs** | **Concentration (mg/mL)** | **Dosage (mg/kg)** | **Preparation method** | **Storage condition** | **Formulation frequency** |
|---|---|---|---|---|---|
| hIgG1 control | 1 | 10 | The solution was diluted with normal saline for injection. | 4°C | For immediate use after preparation |
| JMT601 | 1 | 10 | At the first formulation, aliquots were prepared to match the required dose per use. Repeated freezing and thawing should be avoided. 0.52 mL 20 mg/ml JMT601 was taken and 9.88 mL normal saline for injection was added thereto, thereby obtaining 10.4 ml 1mg/ml JMT601 solution for single use. | 4°C | For immediate use after preparation |
| JMT601 | 0.33 | 3.3 | 3.168 mL 1mg/ml JMT601 was taken and 6.432 mL normal saline for injection was added thereto, thereby obtaining 9.6 ml 0.33 mg/ml JMT601 solution for single use. | 4°C | For immediate use after preparation |
| Rituximab | 1 | 10 | 0.96 mL 10mg/ml rituximab was taken and 8.64 mL normal saline for injection was added thereto, thereby obtaining 9.6 ml 1mg/ml rituximab solution for single use. | 4°C | For immediate use after preparation |
| Bendamustine (salt factor:1.102) | 3 | 30 | 15.87 mg bendamustine (actual drug content: 14.4 mg) was weighed, 4.8 ml water for injection was added, and then the mixture was shaken and mixed well to obtain a medical solution of 3 mg/ml, which was used after filtration. | 4°C | For immediate use after preparation |

### 2.3 Observation

The experimental protocols for animal experiments in the course of this experiment were reviewed and approved by the CrownBio IACUC Committee. During the experiment, animal experiments were performed in accordance with the requirements of AAALAC. After tumor inoculation, routine monitoring included tumor growth and the effects of treatment on the normal behavior of the animals, specifically including the activity, food and water intake, weight gain or loss, eyes, coat and other abnormalities of the experimental animals. The clinical symptoms observed during the experiment were recorded in the raw data.

The data were collected in the experiment using StudyDirector^{™} (version 3.1.399.19, provided by Studylog System,Inc., S.San Francisco, CA, USA) software, including measurements of the long and short diameters of the tumor and the weighing of the animal body weight.

The experiment was terminated after 21 days of dosing by group. The tumor-bearing mice were photographed and the tumors were weighed and then photographed.

### 2.4 Data Analysis

Different treatment groups were compared, and homogeneity of variances was confirmed while one way ANOVA was performed. If the variances were homogeneous, Dunnnett's test would continue to be used for pairwise comparisons. If the variances were not homogeneous, the non-parametric Kruskal wallis test would be used instead of one way ANOVA, and Dunn's test would be used for pairwise comparisons. All statistical analyses and graphic plotting were performed using GraphPad Prism 8.4.2, and p values less than 0.05 were considered statistically significant.

### 3. Experimental Results

The average tumor volume of mice in the 10 mg/kg IgG1 control group was 1629.39 mm³ on day 21 after dosing by group, and the tumor volume doubling time was 6 days. Day 21 after dosing by group was used as the evaluation point for drug treatment effect, and the relative tumor volume of mice in the test group and the control group was compared to see if there was any significant difference. On day 21 after dosing by group, the average tumor volume of mice in the 10 mg/kg rituximab group was 1121.67 mm³, and the tumor inhibition rate was 33.39%, which had no significant difference compared with that of the control group (p value>0.9999), and the tumor volume doubling time was 8 days. The average tumor volume of mice in the 3.3 mg/kg JMT601 group was 825.07 mm³, and the tumor inhibition rate was 50.00%, which had no significant difference compared with that of the control group (p value>0.9999), and the tumor volume doubling time was 9 days. The average tumor volume of mice in the 10 mg/kg JMT601 group was 264.67 mm³, and the tumor inhibition rate was 84.69%, which was significantly different from that of the control group (p value was 0.0002), and the tumor volume doubling time was 27 days (calculated from existing data). The average tumor volume of mice in the 30 mg/kg bendamustine group was 945.10 mm³, and the tumor inhibition rate was 44.00%, which had no significant difference compared with that of the control group (p value>0.9999), and the tumor volume doubling time was 8 days. The average tumor volume of mice in the 30 mg/kg bendamustine in combination with 3.3 mg/kg JMT601 group was 178.85 mm³, and the tumor inhibition rate was 89.02%, which was significantly different from that of the control group (p value <0.0001), and the tumor volume doubling time was 88 days (estimated from existing data).

The analysis results of tumor weight were basically consistent with those of relative tumor volume.

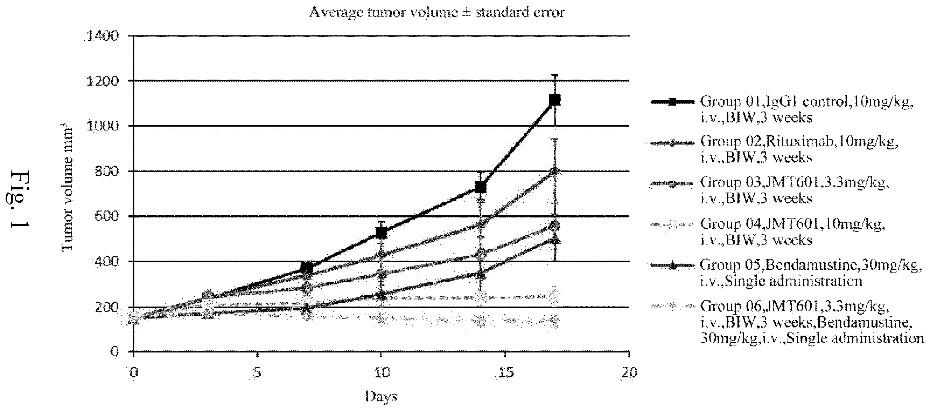

The tumor growth inhibition of mice in each treatment group and control group was shown in Table 4 and FIG. 1.

**Table 4: Antitumor effect of test drugs in a WSU-DLCL2 human lymphoma subcutaneous model**

| **Group** | **Treatment** | **Tumor volume on the day of grouping ³ (mm³)** | **Tumor volume on day 21 after dosing by group^{a} (mm³)^{b}** | **RTV** | **T/C (%)** | **TGI (%)** | **Tumor weight (mg)^{a, b}** | **PR^{c}** | **CR^{d}** | **TTD^{e} (day)** |
|---|---|---|---|---|---|---|---|---|---|---|
| 1 | IgG1 control (10 mg/kg) | 150.60±8.93 | 1629.39±165.76 | 10.84 | - | - | 1730.28±173.30 | 0 | 0 | 6 |
| 2 | Rituximab (10 mg/kg) | 150.50±9.75 | 1121.67±200.03 | 7.22 | 66.61 | 33.39 | 1172.09±223.62 | 0 | 0 | 8 |
| 3 | JMT601 (3.3 mg/kg) | 150.73±9.00 | 825.07±152.06 | 5.42 | 50.00 | 50.00 | 819.96±159.51 | 0 | 0 | 9 |
| 4 | JMT601 (10 mg/kg) | 150.68±10.33 | 264.67±58.77^{***} | 1.66 | 15.31 | 84.69 | 192.53±54.56^{***} | 0 | 0 | 27 |
| 5 | Bendamustine (30 mg/kg) | 150.45±10.63 | 945.10±176.69 | 6.07 | 56.00 | 44.00 | 921.74±198.58 | 0 | 0 | 8 |
| 6 | JMT601 (3.3 mg/kg) | 150.43±6.61 | 178.85±55.56^{***}Δ | 1.19 | 10.98 | 89.02 | 169.39±56.98^{***} | 2/8 | 0 | 88 |
| | Bendamustine (30 mg/kg) | | | | | | | | | |

| | | | | | | | | | | |
|---|---|---|---|---|---|---|---|---|---|---|
| Note: a. The data were expressed as "mean±standard error"; b: vs group-1, *represented p<0.05, **represented p < 0.01, ***represented p < 0.001; vs group 5, Δrepresented p<0.05. c: PR: partial remission; d: CR: complete remission. e: TTD: tumor volume doubling time. | | | | | | | | | | |

Based on the above experimental results, it can be seen that compared with the use of JMT601 (3.3 mg/kg) and bendamustine (10 mg/kg) alone, the use of JMT601(3.3 mg/kg) in combination with bendamustine (10 mg/kg) can greatly improve the tumor inhibition rate, and has a significant synergistic effect and can achieve an unexpected technical effect.

### Example 2: Results and discussion of safety studies of test drugs in WSU-DLCL2 human lymphoma subcutaneous transplant model

In this experiment, one mouse (4174#) in the group of 3.3 mg/kg JMT601 in combination with 30 mg/kg bendamustine had a body weight loss greater than 20% on days 15, 18 and 19 after dosing by group, reaching the humane endpoint and was euthanized. In all mice that exhibited weight loss, it was found that the loss was attributable to reduced appetite, and no significant abnormalities were found in the necropsy results. It is speculated that the weight loss was caused by individual differences in mice. All the tested drugs were well tolerated by other mice in each group.

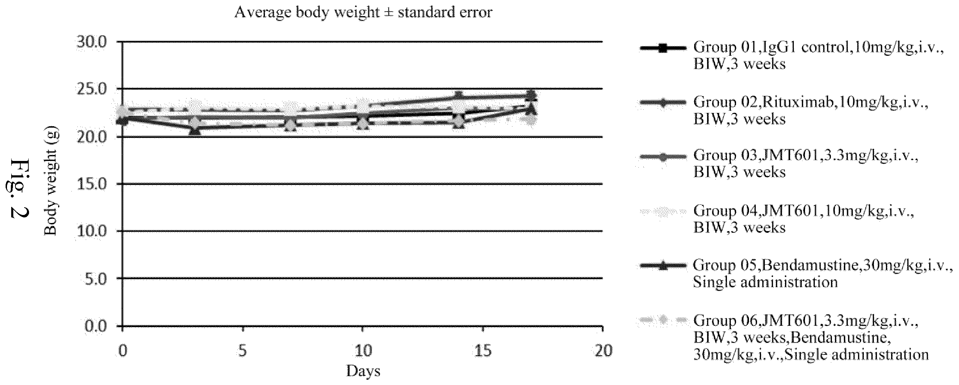

The body weight changes in the treatment group and the control group after dosing were shown in Table 5 and Figure 2.

**Table 5: Drug efficacy data**

| **Group** | **Treatment** | **Number of animals at the start/end of the experiment** | **Mean body weight g (*x̅*±S)** | | **Body weight change rate (%)** |
|---|---|---|---|---|---|
| | | | **Day 0 after grouping** | **Day 21 after grouping** | **Day 21 after grouping** |
| 1 | IgG1 control (10 mg/kg) | 8/8 | 22.0±0.6 | 23.5±0.6 | +7.04 |
| 2 | Rituximab (10 mg/kg) | 8/8 | 22.8±0.4 | 24.5±0.4 | +7.34 |
| 3 | JMT601 (3.3 mg/kg) | 8/8 | 21.9±0.7 | 23.8±0.7 | +8.71 |
| 4 | JMT601 (10 mg/kg) | 8/8 | 22.5±0.7 | 23.4±0.5 | +4.45 |
| 5 | Bendamustine (30 mg/kg) | 8/8 | 22.0±0.2 | 23.2±0.5 | +5.33 |
| 6 | JMT601 (3.3 mg/kg) | 8/7 | 22.6±0.5 | 22.2±0.5 | -0.10 |
| | Bendamustine (30 mg/kg) | | | | |

The above results showed that tumor-bearing mice can tolerate treatment with rituximab (10 mg/kg), JMT601 (3.3 mg/kg and 10 mg/kg), and bendamustine (30 mg/kg). There was no significant difference in tolerability between the combined therapy and the single drug treatment, reflecting the good safety of the combined drug regimen.

The fusion protein-related sequences involved in the present application were as follows:
**SEQ ID NO:1** 
MEWSWVFLFFLSVTTGVHSEVQLVESGGGLVQPGRSLRLSCAASGFTFNDYAMHWVRQA
PGKGLEWVSTISWNSGSIGYADSVKGRFTISRDNAKKSLYLQMNSLRAEDTALYYCAKDIQ
YGNYYYGMDVWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISK
AKGQPREPQVYTLPPSRDELTKNQVSLSCAVKGFYPSDIAVEWESNGQPENNYKTTPPVLD
SDGSFFLVSKLTVDKSRWQQGNVFSCSVMHEALHNHYTQKSLSLSPGK
**SEQ ID NO:2** 
MSVPTQVLGLLLLWLTDARCEIVLTQSPATLSLSPGERATLSCRASQSVSSYLAWYQQKPG
QAPRLLIYDASNRATGIPARFSGSGSGTDFTLTISSLEPEDFAVYYCQQRSNWPITFGQGTRL
EIKRTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
**SEQ ID NO:3** (the position of the N80A mutation was indicated by the underlined and bold "A") 
MEWSWVFLFFLSVTTGVHSEEELQVIQPDKSVSVAAGESAILHCTVTSLIPVGPIQWFRGAG
PARELIYNQKEGHFPRVTTVSESTKRENMDFSISISAITPADAGTYYCVKFRKGSPDTEFKSG
AGTELSVRAKPDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCKVSNKALP
APIEKTISKAKGQPREPQVYTLPPSRDELTKNQVSLWCLVKGFYPSDIAVEWESNGQPENN
YKTTPPVLDSDGSFFLYSKLTVDKSRWQQGNVFSCSVMHEALHNHYTQKSLSLSPGK
**SEQ ID NO:4** MEWSWVFLFFLSVTTGVHSQVQLVQSGAEVKKPGSSVKVSCKASGYAFSYSWINWVRQA 
PGQGLEWMGRIFPGDGDTDYNGKFKGRVTITADKSTSTAYMELSSLRSEDTAVYYCARNV
FDGYWLVYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDKKVEPKSC
DKTHTCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISKAKG
QPREPQVYTLPPSRDELTKNQVSLSCAVKGFYPSDIAVEWESNGQPENNYKTTPPVLDSDG
SFFLVSKLTVDKSRWQQGNVFSCSVMHEALHNHYTQKSLSLSPGK
**SEQ ID NO:5** 
MSVPTQVLGLLLLWLTDARCDIVMTQTPLSLPVTPGEPASISCRSSKSLLHSNGITYLYWYL
QKPGQSPQLLIYQMSNLVSGVPDRFSGSGSGTDFTLKISRVEAEDVGVYYCAQNLELPYTF
GGGTKVEIKRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGEC
**SEQ ID NO:6** (the underlined and bold "N" was the wild-type D1 domain residue) 
MEWSWVFLFFLSVTTGVHSEEELQVIQPDKSVSVAAGESAILHCTVTSLIPVGPIQWFRGAG
PARELIYNQKEGHFPRVTTVSESTKRENMDFSISISNITPADAGTYYCVKFRKGSPDTEFKSG
AGTELSVRAKPDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCKVSNKALP
APIEKTISKAKGQPREPQVYTLPPSRDELTKNQVSLWCLVKGFYPSDIAVEWESNGQPENN
YKTTPPVLDSDGSFFLYSKLTVDKSRWQQGNVFSCSVMHEALHNHYTQKSLSLSPGK
Note: The underlined part was the signal peptide sequence, which can be replaced as needed.

Although specific embodiments of the present application have been described in detail, those skilled in the art will understand that, based on all the teachings disclosed, various modifications and substitutions may be made to those details, and these changes are within the protection scope of the present application. The full scope of the application is defined by the appended claims and any equivalents thereof.

## Claims

1. A pharmaceutical composition for treating cancer, comprising a CD20/CD47 double-blocking bifunctional fusion protein and a bifunctional alkylating agent.

2. The pharmaceutical composition according to claim 1, wherein:
the functional portion that blocks CD20 in the CD20/CD47 double-blocking bifunctional fusion protein is an antibody or antigen-binding portion thereof against CD20; and/or
the functional portion that blocks CD47 in the CD20/CD47 double-blocking bifunctional fusion protein is an antibody or antigen-binding portion thereof against CD47, and/or a natural ligand (e.g., SIRPα protein) of CD47 or a ligand active moiety derived from the natural ligand (e.g., the SIRPα protein);
optionally, the functional portion that blocks CD20 and the functional portion that blocks CD47 each comprises an Fc region, such as a wild-type Fc region or an Fc region with mutations based on Knobs-into-holes technology.

3. The pharmaceutical composition according to claim 2, wherein the ligand active moiety is an entire extracellular domain of the SIRPα protein, an extracellular truncation of the SIRPα protein, or a non-high affinity mutant thereof.

4. The pharmaceutical composition according to any one of claims 1-3, wherein the CD20/CD47 double-blocking bifunctional fusion protein is a fusion protein comprising a first arm that targets CD20 with high affinity (e.g., a first arm in the form of an antibody) and a second arm that blocks the interaction of CD47 and SIRPα with low affinity (e.g., a second arm in the form of a natural ligand of CD47 or a ligand active moiety derived from the natural ligand); preferably, the binding affinity of the fusion protein to the CD20 protein is at least 6 times that of the fusion protein to the CD47 protein.

5. The pharmaceutical composition according to claim 4, wherein:
the first arm comprises the amino acid sequence shown at positions 20-471 of SEQ ID NO: 1 and the amino acid sequence shown at positions 21-234 of SEQ ID NO: 2, and the second arm comprises the amino acid sequence shown at positions 20-363 of SEQ ID NO: 3 or 6; or
the first arm comprises the amino acid sequence shown at positions 20-244 of SEQ ID NO: 1 and the amino acid sequence shown at positions 21-234 of SEQ ID NO: 2, and the second arm comprises the amino acid sequence shown at positions 20-136 of SEQ ID NO: 3 or 6; or
the first arm comprises the amino acid sequence shown at positions 20-468 of SEQ ID NO:4 and the amino acid sequence shown at positions 21-239 of SEQ ID NO:5, and the second arm comprises the amino acid sequence shown at positions 20-363 of SEQ ID NO:3 or 6; or
the first arm comprises the amino acid sequence shown at positions 20-241 of SEQ ID NO:4 and the amino acid sequence shown at positions 21-239 of SEQ ID NO:5, and the second arm comprises the amino acid sequence shown at positions 20-136 of SEQ ID NO:3 or 6.

6. The pharmaceutical composition according to claim 4, wherein the first arm comprises the amino acid sequences as set forth in SEQ ID NO: 1 and SEQ ID NO: 2, and the second arm comprises the amino acid sequence as set forth in SEQ ID NO: 3.

7. The pharmaceutical composition according to claim 4, wherein the first arm consists of the amino acid sequences as set forth in SEQ ID NO: 1 and SEQ ID NO: 2, and the second arm consists of the amino acid sequence as set forth in SEQ ID NO: 3.

8. The pharmaceutical composition according to any one of claims 1-7, wherein the bifunctional alkylating agent is a bifunctional derivative of nitrogen mustard, for example, bendamustine or a pharmaceutically acceptable salt, solvate or prodrug thereof; chlorambucil; or L-phenylalanine mustard; preferably bendamustine or a pharmaceutically acceptable salt, solvate or prodrug thereof.

9. The pharmaceutical composition according to claim 8, wherein the bifunctional alkylating agent is bendamustine hydrochloride.

10. The pharmaceutical composition according to any one of claims 1-9, wherein the cancer is selected from lymphoma, breast cancer, colorectal cancer, lung cancer, pancreatic cancer, esophageal cancer, endometrial cancer, ovarian cancer, gastric cancer, prostate cancer, kidney cancer, cervical cancer, myeloma, leukemia, thyroid cancer, uterine cancer, bladder cancer, neuroendocrine cancer, head and neck cancer, liver cancer, nasopharyngeal cancer, testicular cancer, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma or myelodysplastic syndrome; preferably lymphoma which includes Hodgkin lymphoma and non-Hodgkin lymphoma; more preferably non-Hodgkin lymphoma which includes diffuse large B-cell lymphoma, follicular lymphoma, mantle cell lymphoma, and mucosa-associated lymphoid tissue lymphoma; and most preferably diffuse large B-cell lymphoma.

11. The pharmaceutical composition according to any one of claims 1-10, wherein the cancer is a CD20-positive cancer, for example a CD20-positive non-Hodgkin lymphoma (such as diffuse large B-cell lymphoma).

12. Use of a CD20/CD47 double-blocking bifunctional fusion protein and a bifunctional alkylating agent in the manufacture of a pharmaceutical composition for treating cancer.

13. A combination of a CD20/CD47 double-blocking bifunctional fusion protein and a bifunctional alkylating agent for use in the treatment of cancer.

14. A method of treating cancer in an individual, the method comprising administering to the individual in need thereof a therapeutically effective amount of a CD20/CD47 double-blocking bifunctional fusion protein and a bifunctional alkylating agent.

15. The use according to claim 12 or the combination according to claim 13 or the method according to claim 14, wherein the bifunctional alkylating agent is a bifunctional derivative of nitrogen mustard, for example, bendamustine or a pharmaceutically acceptable salt, solvate or prodrug thereof; chlorambucil; or L-phenylalanine mustard; preferably bendamustine or a pharmaceutically acceptable salt, solvate or prodrug thereof; more preferably bendamustine hydrochloride.

16. The use or combination or method according to any one of claims 12-15, wherein:
the functional portion that blocks CD20 in the CD20/CD47 double-blocking bifunctional fusion protein is an antibody or antigen-binding portion thereof against CD20; and/or
the functional portion that blocks CD47 in the CD20/CD47 double-blocking bifunctional fusion protein is an antibody or antigen-binding portion thereof against CD47, and/or a natural ligand (e.g., SIRPα protein) of CD47 or a ligand active moiety derived from the natural ligand (e.g., the SIRPα protein);
optionally, the functional portion that blocks CD20 and the functional portion that blocks CD47 each comprises an Fc region, such as a wild-type Fc region or an Fc region with mutations based on Knobs-into-holes technology.

17. The use or combination or method according to claim 16, wherein the ligand active moiety is the entire extracellular domain of the SIRPα protein, an extracellular truncation of the SIRPα protein, or a non-high affinity mutant thereof.

18. The use or combination or method according to any one of claims 12-17, wherein the CD20/CD47 double-blocking bifunctional fusion protein is a fusion protein comprising a first arm that targets CD20 with high affinity (e.g., a first arm in the form of an antibody) and a second arm that blocks the interaction of CD47 and SIRPα with low affinity (e.g., a second arm in the form of a natural ligand of CD47 or a ligand active moiety derived from the natural ligand); preferably, the binding affinity of the fusion protein to the CD20 protein is at least 6 times that of the fusion protein to the CD47 protein.

19. The use or combination or method according to claim 18, wherein:
the first arm comprises the amino acid sequence shown at positions 20-471 of SEQ ID NO: 1 and the amino acid sequence shown at positions 21-234 of SEQ ID NO: 2, and the second arm comprises the amino acid sequence shown at positions 20-363 of SEQ ID NO: 3 or 6; or
the first arm comprises the amino acid sequence shown at positions 20-244 of SEQ ID NO: 1 and the amino acid sequence shown at positions 21-234 of SEQ ID NO: 2, and the second arm comprises the amino acid sequence shown at positions 20-136 of SEQ ID NO: 3 or 6; or
the first arm comprises the amino acid sequence shown at positions 20-468 of SEQ ID NO:4 and the amino acid sequence shown at positions 21-239 of SEQ ID NO:5, and the second arm comprises the amino acid sequence shown at positions 20-363 of SEQ ID NO:3 or 6; or
the first arm comprises the amino acid sequence shown at positions 20-241 of SEQ ID NO:4 and the amino acid sequence shown at positions 21-239 of SEQ ID NO:5, and the second arm comprises the amino acid sequence shown at positions 20-136 of SEQ ID NO:3 or 6.

20. The use or combination or method according to claim 18, wherein the first arm comprises the amino acid sequences as set forth in SEQ ID NO: 1 and SEQ ID NO: 2, and the second arm comprises the amino acid sequence as set forth in SEQ ID NO: 3.

21. The use or combination or method according to claim 18, wherein the first arm consists of the amino acid sequences as set forth in SEQ ID NO: 1 and SEQ ID NO: 2, and the second arm consists of the amino acid sequence as set forth in SEQ ID NO: 3.

22. The use or combination or method according to any one of claims 12-21, wherein the cancer is selected from lymphoma, breast cancer, colorectal cancer, lung cancer, pancreatic cancer, esophageal cancer, endometrial cancer, ovarian cancer, gastric cancer, prostate cancer, kidney cancer, cervical cancer, myeloma, leukemia, thyroid cancer, uterine cancer, bladder cancer, neuroendocrine cancer, head and neck cancer, liver cancer, nasopharyngeal cancer, testicular cancer, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma or myelodysplastic syndrome; preferably lymphoma which includes Hodgkin lymphoma and non-Hodgkin lymphoma; more preferably non-Hodgkin lymphoma which includes diffuse large B-cell lymphoma, follicular lymphoma, mantle cell lymphoma, and mucosa-associated lymphoid tissue lymphoma; and most preferably diffuse large B-cell lymphoma.

23. The use or combination or method according to any one of claims 12-22, wherein the cancer is a CD20-positive cancer, for example a CD20-positive non-Hodgkin lymphoma (such as diffuse large B-cell lymphoma).

24. The use or combination or method according to any one of claims 12-23, wherein the CD20/CD47 double-blocking bifunctional fusion protein and the bifunctional alkylating agent are formulated together or physically separate.

25. The use or combination or method according to any one of claims 12-23, wherein the CD20/CD47 double-blocking bifunctional fusion protein is administered before, simultaneously with or after the bifunctional alkylating agent.

26. The use or combination or method according to any one of claims 12-25, wherein the bifunctional fusion protein is administered at a dose of 0.3 mg/kg-20 mg/kg, for example, 0.5 mg/kg, 2 mg/kg, 5 mg/kg, 10 mg/kg or 15 mg/kg.

27. The use or combination or method according to any one of claims 12-26, wherein the bifunctional alkylating agent is administered at a dose of 5 mg/kg-40 mg/kg, for example, 5 mg/kg, 10 mg/kg, 20 mg/kg, 30 mg/kg or 40 mg/kg.

28. The use or combination or method according to any one of claims 12-27, wherein the administration frequencies of the CD20/CD47 double-blocking bifunctional fusion protein and the bifunctional alkylating agent are independently at least once every 2 weeks, for example, once every 2 weeks, once every 3 weeks or once every 4 weeks, preferably once every 2 weeks or once every 3 weeks; or the administration frequencies are independently more than once a week, for example, twice a week.

29. The use or combination or method according to any one of claims 12-28, wherein the administration modes of the CD20/CD47 double-blocking bifunctional fusion protein and the bifunctional alkylating agent are independently inhalation, intranasal administration, oral administration, intravenous injection, subcutaneous injection or intramuscular injection.
